# EUROPÄISCHE PATENTANMELDUNG

(11) **EP 1 985 331 A1**
(43) Veröffentlichungstag der Anmeldung: **29.10.2008**
(21) Anmeldenummer: 08154868.7
(22) Anmeldetag: 21.04.2008
(51) Int. Cl.: A61M 39/20, A61M 39/22, F16K 11/08

(54) **Dreiwegehahn für medizinische Anwendungen**

(30) Priorität: 25.04.2007 DE 202007005897 U
(71) Anmelder: Schwöbel, Eckhard, 6005 Luzern (CH)
(72) Erfinder: Schwöbel, Eckhard, 6005 Luzern (CH)
(74) Vertreter: Selting, Günther

(57) **Zusammenfassung**

Ein Dreiwegehahn weist ein Ventilgehäuse (10) auf, in dem ein Ventilküken (11) drehbar ist, Von dem Ventilgehäuse (10) stehen durchgehende Anschlüsse (12, 14) in koaxialer Ausrichtung ab, während ein seitlicher Anschluss (16) abgeht. Der seitliche Anschluss (16) kann mit einer Kappe (17) verschlossen werden. Er bildet einen Zuspritzanschluss. Um beim Zuspritzen eines Medikaments in den Dreiwegehahn die Kappe (17) definiert anzubringen, ist an dem mit dem Ventilküken (11) verbundenen Griff (21) ein erstes Halteelement (31) vorgesehen, das mit einem zweiten Halteelement (32) der Kappe (17) zusammengreifen kann. Daher kann die Kappe (17) vorübergehend auf dem Griff (21) fixiert werden. Verhindert wird, dass die Kappe (17) herunterfallen oder verlegt wird.

## Beschreibung

Die Erfindung betrifft einen Dreiwegehahn für medizinische Anwendungen, mit einem Ventilgehäuse, das drei Anschlüsse aufweist, einem in dem Ventilgehäuse drehbaren Ventilküken mit einem Griff, wobei durch Drehen des Griffs um die Achse des Ventilkükens eine Auswahl der miteinander zu verbindenden und der voneinander zu isolierenden Anschlüsse erfolgt, und mit einer Kappe zum Verschließen eines der Anschlüsse.

Dreiwegehähne werden in der Medizintechnik für verschiedenartige Anwendungen benötigt. Eine dieser Anwendungen sind Infusionssysteme, bei denen eine Infusionsflüssigkeit aus einem Infusionsbehälter durch einen Schlauch einem Patienten zugeführt wird. Im Verlauf des Schlauchs sind eine Tropfkammer und eine Schlauchklemme oder ein anderes Dosierorgan zur Drosselung des Flüssigkeitsflusses angeordnet. Zum Zugeben eines Medikaments in die Infusionsflüssigkeit wird ein Dreiwegehahn benutzt. Dieser weist einen ersten Anschluss für die ankommende Infusionsflüssigkeit, einen zweiten Anschluss für die abgehende Infusionsflüssigkeit und einen dritten Anschluss für das Zuspritzen eines Medikaments auf. Der Dreiwegehahn hat ein zylindrisches Ventilgehäuse, in dem ein drehbares Ventilküken angeordnet ist. Die Verstellung des Ventils erfolgt durch Drehen des Ventilkükens, Der Dreiwegehahn erlaubt eine Durchgangsposition, in der die koaxialen Anschlüsse miteinander verbunden sind, eine Absperrposition, in der die koaxialen Anschlüsse voneinander getrennt sind und eine Zuspritzposition, in der ein seitlicher Anschluss mit einem der axialen Anschlüsse verbunden ist, während der andere axiale Anschluss abgetrennt ist.

Beim Anlegen des Infusionssystems wird von dem Zulaufanschluss des Dreiwegehahns eine erste Kappe abgenommen und beiseite gelegt. Wenn ein Medikament in das Infusionssystem eingespritzt werden soll, wird der seitliche Anschluss des Dreiwegehahns geöffnet, indem eine weitere Kappe von diesem Anschluss abgeschraubt und verworfen wird. Dann wird das Ventilküken auf Zuspritzen eingestellt, wobei der den Zuspritzanschluss bildende seitliche Anschluss mit einem der beiden Hauptanschlüsse verbunden wird. Nach dem Zuspritzen einer Flüssigkeit in den seitlichen Anschluss wird die Spritze wieder abgenommen und der seitliche Anschluss wird durch erneutes Aufsetzen der ersten Kappe verschlossen.

Bei solchen Manipulationen kann es vorkommen, dass die erste Kappe, die ein Losteil bildet, auf den Boden fällt oder dass die Kappe von dem medizinischen Personal beiseite gelegt und anschließend nicht wiedergefunden wird. Eine heruntergefallene Kappe stellt ein erhöhtes Kontaminationsrisiko dar.

Andererseits muss der seitliche Anschluss nach seiner Benutzung wieder verschlossen werden.

Der Erfindung liegt die Aufgabe zugrunde, einen Dreiwegehahn mit Kappe zum Verschließen mindestens eines der Anschlüsse zu schaffen, der eine Handhabung ermöglicht, welche das Herunterfallen oder Verlegen der Kappe ausschließt.

Der erfindungsgemäße Dreiwegehahn ist durch den Patentanspruch 1 definiert. Er ist dadurch gekennzeichnet, dass an dem Griff oder am Ventilgehäuse ein erstes Halteelement vorgesehen ist und dass an der Kappe ein mit dem ersten Halteelement lösbar zusammengreifendes zweites Halteelement vorgesehen ist.

Die Halteelemente bewirken, dass die Kappe, die von einem Anschluss abgenommen wurde, an einer anderen Stelle des Dreiwegehahns vorübergehend sicher deponiert werden kann. Der Dreiwegehahn weist daher eine Halteeinrichtung zur Befestigung der Kappe während des Nichtgebrauchs der Kappe auf. Das medizinische Personal nimmt die Kappe von dem betreffenden Anschluss ab und bringt sie in eine definierte Aufbewahrungsstellung, wobei das zweite Halteelement der Kappe mit dem ersten Halteelement zusammengesteckt wird. Das erste Halteelement befindet sich vorzugsweise an dem Griff. Andere Möglichkeiten der Positionierung des ersten Halteelements sind das Ventilgehäuse bzw. das Ventilküken. In jedem Fall wird eine definierte Haltevorrichtung zum geordneten Festhalten der Kappe geschaffen.

Gemäß einer bevorzugten Ausgestaltung der Erfindung ist vorgesehen, dass der Griff eine Nabe aufweist, an der das erste Halteelement vorgesehen ist. Demnach bildet der Griff einen Träger für die Kappe in deren Aufbewahrungsposition.

Gemäß einer bevorzugten Ausgestaltung der Erfindung ist das erste Halteelement koaxial am Ende der Nabe angeordnet, derart, dass die Kappe koaxial auf die Nabe steckbar ist. Alternativ hierzu besteht die Möglichkeit, das erste Halteelement an der dem Griff abgewandten Rückseite anzuordnen und zwar entweder am Ventilgehäuse oder an dem Ventilküken.

Die Halteelemente sind zweckmäßigerweise Rastelemente, so dass die Kappe rastend in die Warteposition eingesetzt wird und aus dieser ausrastend wieder entfernt werden kann. Das an der Kappe vorgesehene Halteelement ist zweckmäßigerweise an der Innenseite eines rohrförmigen Mantels der Kappe angeordnet. Das Halteelement kann an der Innenseite des Mantels einen Wulst oder eine Vertiefung bilden. Es muss sich nicht kontinuierlich um den Umfang der Kappe erstrecken, sondern kann auch aus umfangsmäßig voneinander getrennten Abschnitten bestehen.

Es ist auch möglich, dass die Halteelemente Klemmelemente sind, sind, die nicht-rastend zusammengeklemmt werden.

Im Folgenden wird unter Bezugnahme auf die Zeichnungen ein Ausführungsbeispiel der Erfindung näher erläutert.

Es zeigen:
- Fig. 1: eine Frontansicht des Dreiwegehahns mit Blick auf den Griff,
- Fig. 2: eine Seitenansicht aus Richtung des Pfeiles II von Figur 1 und
- Fig. 3: in ähnlicher Darstellung wie Figur 2 den Zustand mit auf dem Griff befestigter Kappe.

Der Dreiwegehahn weist ein Ventilgehäuse 10 mit einer im wesentlichen zylindrischen Bohrung auf. Darin befindet sich ein generell zylindrisches Ventilküken 11, das um seine Achse gedreht werden kann.

Von dem Ventilgehäuse 10 steht ein erster Anschluss 12 ab, der den Zulaufanschluss bildet. Dieser ist hier mit einer Kappe 13 verschlossen. Der Anschluss 12 und die sterile Kappe 13 bilden eine Luer-Lokverbindung, so dass die Kappe 13 abgeschraubt werden kann.

In axialer Ausrichtung mit dem ersten Anschluss 12 ist ein zweiter Anschluss 14 vorgesehen, der in entgegengesetzte Richtung von dem Ventilgehäuse 10 absteht. Der Anschluss 14 weist eine Drehmuffe 15 als männlicher Luer-Konus auf. Der Anschluss 14 bildet einen Ablaufanschluss.

Von dem Ventilgehäuse 10 steht ferner ein seitlicher Anschluss 16 ab, welcher den Zuspritzanschluss bildet. Dieser ist mit einer Kappe 17 verschlossen. Die Kappe 17 bildet einen männlichen Luer-Konus. Sie weist einen zylindrischen Mantel 18 mit Innengewinde 19 auf. Ein konischer Ansatz 20 dringt in die konische Bohrung des Anschlusses 16 ein, wenn die Kappe 17 auf diesen aufgeschraubt ist.

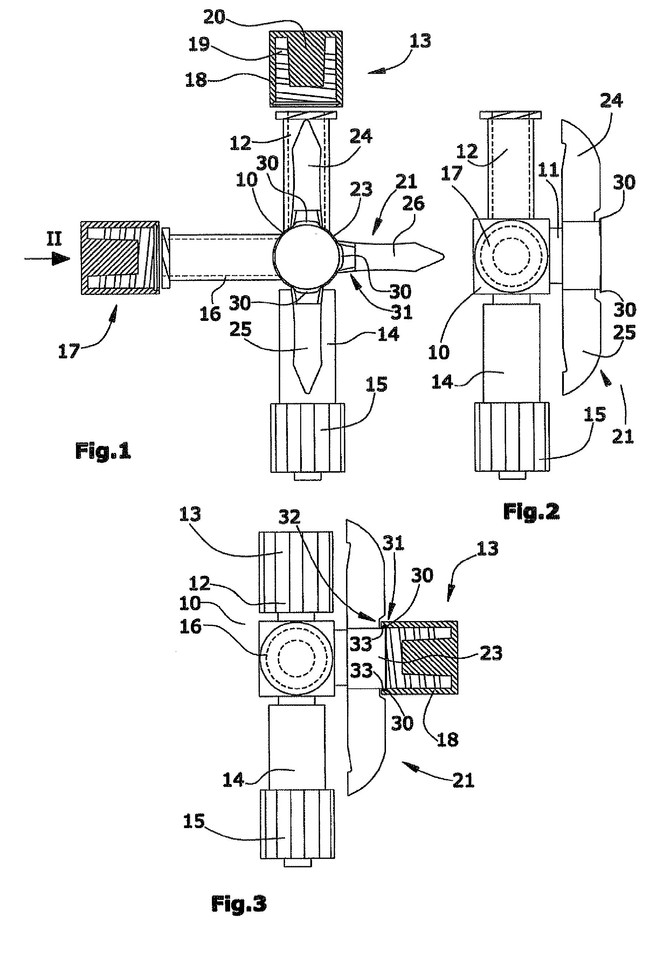

Das Ventilküken 11 ist mit einem Griff 21 verbunden, der als Drehgriff ausgebildet ist, welcher um die Längsachse des Kükens gedreht werden kann. Der Griff 21 weist eine mit dem Ventilküken 11 verbundene Nabe 23 und radial davon abstehende Arme 24, 25, 26 auf. Die Arme 24, 25 befinden sich in gegenseitiger Ausrichtung, während der Arm 26 seitlich davon absteht. Durch die Stellung der Arme relativ zu den Anschlüssen 12, 14, 16 ist die Funktionsstellung des Dreiwegehahns sichtbar. In derjenigen Stellung, die der Griff 21 in Figur 1 einnimmt, sind die Anschlüsse 12, 14 untereinander verbunden, während der Anschluss 16 von den anderen Anschlüssen isoliert ist. Würde der Griff 21 um 90° im Uhrzeigersinn gedreht, so wären die Anschlüsse 16 und 14 verbunden, während der Anschluss 12 abgetrennt wäre. Würde der Griff um weitere 90° gedreht, wären alle Anschlüsse 12, 14, 16 untereinander verbunden. Würde der Griff 21 aus der Stellung in Figur 1 um 90° im Gegenuhrzeigersinn gedreht, so wären die Anschlüsse 12 und 16 verbunden, während der Anschluss 14 abgetrennt wäre.

Die Nabe 23 ist rohrförmig ausgebildet. An ihrer Außenseite befinden sich Vorsprünge 30, welche das erste Halteelement 31 bilden. Die Vorsprünge sind um den Umfang verteilt angeordnet. Sie sind im vorliegenden Fall jeweils zu den Armen 24, 25, 26 ausgerichtet.

Zweite Halteelemente 32 befinden sich an der Kappe 13. Es handelt sich um Vorsprünge 33, die von der Innenseite des Mantels 18 nach innen abstehen. Wird die Kappe 13 auf die Nabe 23 aufgedrückt, dann schnappt das zweite Halteelement 32 über das erste Halteelement 31 der Nabe, so dass die Kappe 13 auf dem Griff 21 in definierter Form festgehalten wird, so wie dies in Figur 3 dargestellt ist. Die Kappe 13 kann somit auch leicht ergriffen und wieder von dem Griff abgezogen werden.

Wird zum Öffnen des den Zulauf bildenden Anschlusses 12 die Kappe 13 von diesem abgenommen, so kann sie gemäß Figur 3 auf den Griff 21 aufgedrückt werden, um sicher festgehalten zu werden. Auch ein Drehen des Griffs 21 mit aufgesetzter Kappe 17 ist möglich. Zum Zuspritzen eines Medikaments wird von dem Anschluss 16 die Kappe 17, die unsteril sein kann, entfernt und verworfen. Dann erfolgt durch den seitlichen Anschluss 16 das Zuspritzen. Nach Beendigung des Zuspritzens wird die Kappe 13 von dem Griff 21 abgenommen und nunmehr auf den Anschluss 16 geschraubt, um diesen abdichtend zu verschließen.

Bei dem beschriebenen Beispiel wird diejenige Kappe 13, die den Zulaufanschluss verschließt, an dem ersten Halteelement 31 vorübergehend geparkt und dann auf den seitlichen Anschluss 16 umgesetzt. Es besteht aber auch die Möglichkeit, die Kappe 17 auf dem Halteelement 31 zu parken und anschließend zu dem seitlichen Anschluss 16 zurückzuführen.

## Patentansprüche

1. Dreiwegehahn für medizinische Anwendungen, mit einem Ventilgehäuse (10), das drei Anschlüsse (12, 14, 16) aufweist, einem in dem Ventilgehäuse drehbaren Ventilküken (11) mit einem Griff (21), wobei durch Drehen des Griffs (21) um die Achse des Ventilkükens (11) eine Auswahl der miteinander zu verbindenden und der voneinander zu isolierenden Anschlüsse (12, 14, 16) erfolgt, und mit einer Kappe (13) zum Verschließen eines der Anschlüsse (16),
**dadurch gekennzeichnet,**
**dass** an dem Griff (21), dem Ventilküken (11) oder dem Ventilgehäuse (10) abseits von den Anschlüssen ein erstes Halteelement (31) vorgesehen ist und dass an der Kappe (13) ein mit dem ersten Halteelement lösbar zusammengreifendes, die Kappe (13) in einer inaktiven Position fixierendes zweites Halteelement (32) vorgesehen ist.

2. Dreiwegehahn nach Anspruch 1, **dadurch gekennzeichnet, dass** der Griff (21) eine Nabe (23) aufweist, an der das erste Halteelement (31) vorgesehen ist.

3. Dreiwegehahn nach Anspruch 2, **dadurch gekennzeichnet, dass** das erste Halteelement (31) an dem Ende der Nabe (23) angeordnet ist, derart, dass die Kappe (17) koaxial auf die Nabe (23) steckbar ist.

4. Dreiwegehahn nach einem der Ansprüche 1 - 3, **dadurch gekennzeichnet, dass** die Halteelemente (31, 32) Rastelemente sind.

5. Dreiwegehahn nach einem der Ansprüche 1 - 3, **dadurch gekennzeichnet, dass** die Halteelemente Klemmelemente sind.
